# EUROPEAN PATENT APPLICATION

(11) **EP 2 161 053 A1**
(43) Date of publication of application: **10.03.2010**
(21) Application number: 09251876.0
(22) Date of filing: 24.07.2009
(51) Int. Cl.: A61M 39/26

(54) **Needleless connector with displacement correction**

(30) Priority: 11.08.2008 US 189353
(71) Applicant: Baxter International Inc., Deerfield, IL 60015-4633 (US); Baxter Healthcare S.A., 8152 Glattpark (Opfikon) (CH)
(72) Inventor: Plishka, Michael, Lake Villa, Illinois 60046 (US); Friend, Anthony D., Algonquin, Illinois 60102 (US); Daniels, Lewis E., Wonder Lake, Illinois 60097 (US)
(74) Representative: Dee, Ian Mark

(57) **Abstract**

A needleless connector includes a housing having an inlet and an outlet, a valve disposed in the housing to control flow between the inlet and the outlet, and a gland disposed in the housing between the inlet and the outlet. The gland has a deformable wall defining a space, and at least one reinforcement attached to the deformable wall within the space.

## Description

### Background

This patent is directed to a needleless connector, and, in particular, to a needleless connector with a mechanism for displacement correction.

Needleless connectors have come into widespread use in intravenous (I.V.) administration sets. Needleless connectors replace more traditional technologies, wherein a needle is used to puncture an elastomeric diaphragm or septum. The use of needles and other pointed instruments presents hazards to equipment, patients and healthcare personnel. For example, accidental needle sticks may permit infectious diseases to be transferred from the patient to the healthcare worker.

However, certain needleless connectors may be subject to retrograde flow. That is, when a syringe, blunt cannula or other instrument is inserted into the connector, a volume of fluid is displaced out of the connector and associated set into the patient. This is referred to antegrade flow. However, when the instrument is withdrawn, a similar volume may be drawn into the I.V. set components or the connector from the patient. This is referred to as retrograde flow or reflux.

It is believed by some that retrograde flow can decrease the efficacy of infusion therapy. It is also believed by some that when the blood and/or other fluids flow into the associated catheter, the blood can clot in the catheter.

To oppose the retrograde flow into the connector and associated catheter, methods and devices have been devised that focus on positive displacement or positive pressure. Initially, methods were devised wherein the healthcare worker would continue to flush through a connector/catheter combination and clamp the infusion line as an instrument, a syringe, for example, was removed from the combination, with the intent that this positive pressure flush would limit retrograde flow. However, the complexities of coordination and timing increase the complexity in providing infusion therapy. Over time, devices were devised wherein a positive displacement would occur automatically within the connector when the intruding instrument was withdrawn from the connector, without the involvement of the healthcare worker. Unfortunately, the complexities of the mechanisms designed to achieve automatic positive displacement led, in certain instances, to the same issues of consistency and reliability posed by the manual positive pressure flush method. For example, positive displacement devices that relied upon a structure that bent upon insertion of an instrument into the connector could have unpredictable modes of deformation.

As set forth in more detail below, the present disclosure sets forth an improved assembly embodying advantageous alternatives to the conventional devices and methods discussed above.

### Summary of the Invention

In one aspect, a needleless connector includes a housing having an inlet and an outlet, a valve disposed in the housing to control flow between the inlet and the outlet, and a gland disposed in the housing between the inlet and the outlet. The gland has a deformable wall defining a space, and at least one reinforcement attached to the deformable wall within the space.

Additional aspects of the disclosure are defined by the claims of this patent.

### Brief Description of the Drawings

It is believed that the disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.

Fig. 1 is a perspective view of a needleless connector according to the present disclosure;

Fig. 2 is a cross-sectional view of the needleless connector of Fig. 1 taken along line 2-2;

Fig. 3 is a cross-sectional view of the needleless connector of Fig. 1 taken along line 3-3;

Fig. 4 is a perspective view of a septum to be used in the needleless connector of Fig. 1;

Fig. 5 is a perspective view of an alternative septum;

Fig. 6 is a perspective view of a further alternative septum;

Fig. 7 is a cross-sectional view of an inlet section to be used in the needleless connector of Fig. 1;

Fig. 8 is an end view of the inlet section of Fig. 7;

Fig. 9 is a perspective view of an outlet section to be used in the needleless connector of Fig. 1;

Fig. 10 is an end view of a gland to be used in the needleless connector of Fig. 1;

Fig. 11 is a cross-sectional view of the gland taken along line 11-11 in Fig 10;

Fig. 12 is a cross-sectional view of the gland taken along line 12-12 in Fig. 10;

Fig. 13 is an end view of an alternative gland to be used in the needleless connector such as the connector illustrated in Fig. 1;

Fig. 14 is a cross-sectional view of the gland taken along line 14-14 in Fig. 13;

Fig. 15 is a cross-sectional view of the gland taken along line 15-15 in Fig. 13;

Fig. 16 is a perspective view of a further alternative gland to be used in needleless connectors such as the connector illustrated in Fig. 1;

Fig. 17 is a cross-sectional view of the gland taken along the line 17-17 in Fig. 16;

Fig. 18 is a perspective view of another alternative gland to be used in needleless connectors such as the connector illustrated in Fig. 1;

Fig. 19 is a cross-sectional view of the gland taken along the line 19-19 in Fig. 18;

Fig. 20 is a plan view of an alternative gland with a variant in regard to the groove in the post of the gland, the gland to be used with needleless connectors such as the connector illustrated in Fig. 1;

Fig. 21 is a plan view of a further alternative gland with a variant in regard to the groove in the post of the gland, the gland to be used with needleless connectors such as the connector illustrated in Fig. 1; and

Fig. 22 is a cross-sectional view of an alternative needleless connector similar to that illustrated above.

### Detailed Description of Various Embodiments

Although the following text sets forth a detailed description of different embodiments of the invention, it should be understood that the legal scope of the invention is defined by the words of the claims set forth at the end of this patent. The detailed description is to be construed as exemplary only and does not describe every possible embodiment of the invention since describing every possible embodiment would be impractical, if not impossible. Numerous alternative embodiments could be implemented, using either current technology or technology developed after the filing date of this patent, which would still fall within the scope of the claims defining the invention.

It should also be understood that, unless a term is expressly defined in this patent using the sentence "As used herein, the term '_' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning. Finally, unless a claim element is defined by reciting the word "means" and a function without the recital of any structure, it is not intended that the scope of any claim element be interpreted based on the application of 35 U.S.C. §112, sixth paragraph

Embodiments of a needleless connector according to the present disclosure are discussed herein. A convention has been used where the direction towards the top of the page is generally referred to as "distal," and the direction towards the bottom of the page is generally referred to as "proximal." It will be recognized that use of this convention is intended to simplify reference to the features as illustrated, not to limit the features to a particular orientation in use, for example.

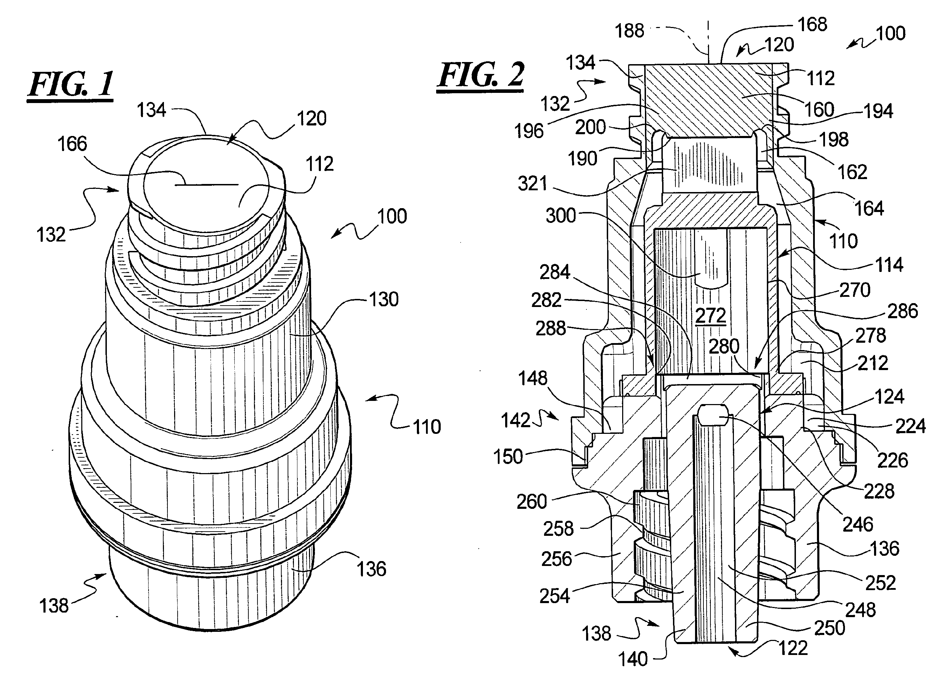

Fig. 1 illustrates an embodiment of a needleless connector 100. The connector 100 may be used to provide multiple fluid connections with a Luer syringe, a blunt cannula or other instrument. The connector 100 may be attached to a conduit in fluid communication with a patient. The conduit may be a peripheral catheter, medical tubing (such as may be used in an intravenous (I.V.) administration set), etc. to form a passageway in fluid communication with the patient for the flow of fluid to the patient. The connector 100 may be used in or connected to other items as well, for example, a vial or vial adapter.

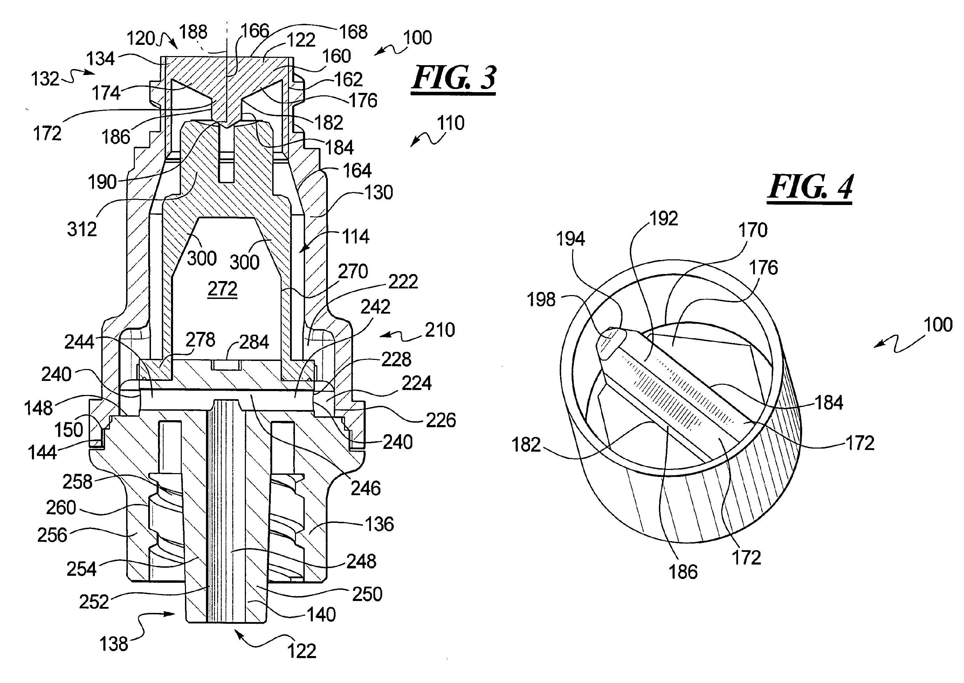

As seen in Figs. 2 and 3, the needleless connector 100 includes a housing 110, a valve 112, and a gland 114. The valve 112 and the gland 114 are disposed in the housing 110. The valve 112 controls the flow of fluid through the housing 110. The gland 114 is intended to minimize retrograde flow into the housing 110 by providing a positive pressure or positive displacement response according to certain embodiments, although neutral (or even negative) displacement may be possible according to other embodiments.

As illustrated in Figs. 2 and 3, the housing 110 has an inlet 120, an outlet 122 and a vent 124. In particular, the housing 110 may include an inlet section 130 with having a distal end 132 with an opening 134 formed therein, the opening 134 defining the inlet 120. The housing 110 may also include an outlet section 136 having a proximal end 138 with an opening 140 formed therein, the outlet section 136 defining the outlet 122.

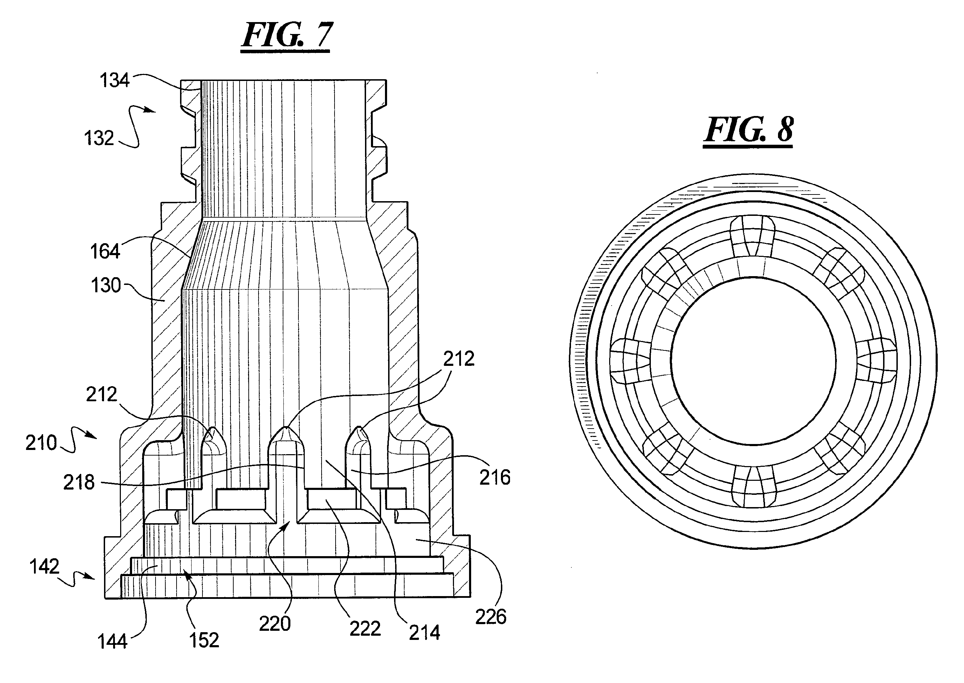

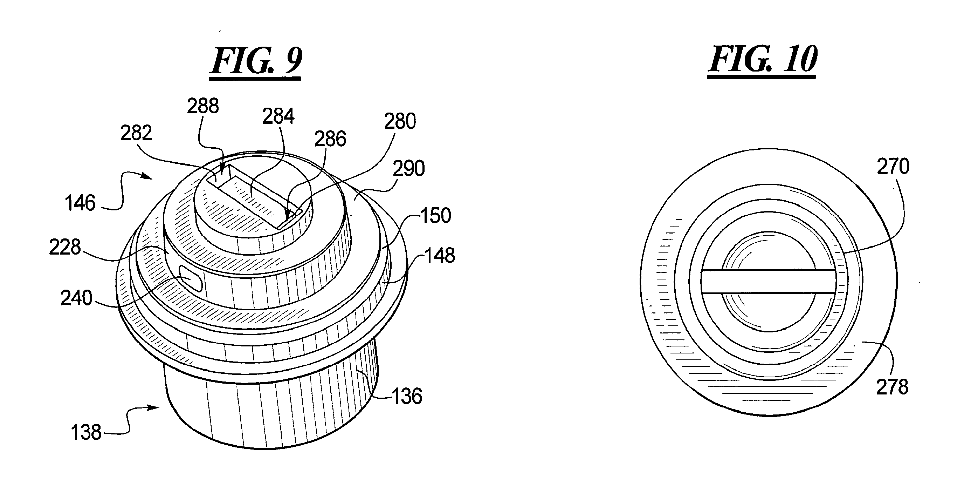

The inlet section 130 is attached to the outlet section 136 with the valve 112 and the gland 114 disposed therein. To this end, the inlet section 130 has a proximal end 142 with an opening 144 formed therein to accept a portion of the outlet section 136 (see Figs. 3 and 7). The outlet section 136, in turn, has a distal end 146 and an outer surface 148 defining a shoulder 150 at the distal end 146 (see Figs. 3 and 9). The distal end 146 of the outlet section 136 is disposed within the opening 144 in the proximal end 142 of the inlet section 130, with a shoulder 152 of the inlet section 130 abutting the shoulder 150 of the outlet section 136. The shoulder 152 may be attached to the shoulder 150 through conventional methods, such as ultrasonic welding.

According to the embodiment illustrated, the valve 112 that controls the flow of fluids between the inlet 120 and the outlet 122 is in the form of an overmolded septum, which may be elastic and resilient and made of silicone, for example. The septum may be molded from a material having a single durometer value, or, alternatively, may be molded in layers with each layer having a different durometer value. According to other embodiments, other valve types may be used, including septums that have not been overmolded, for example. The valve illustrated in the drawings is thus intended to be illustrative and non-limiting.

The illustrated valve 112 includes a first wall 160 that is disposed across the opening 134 in the inlet section 130, and a second wall 162 that depends away from the first wall 160 along a passage 164 formed in the inlet section 130. According to the geometry of the inlet section 130, the first wall 160 is disc-shaped, while the second wall 162 is cylindrical. The first wall 160 of the septum 112 has a slit 166 (see Figs. 1 and 3) that extends either partially or entirely through the first wall 160; if the slit 166 does not extend completely through the septum 112, disposing an instrument through the slit 166 should force the slit 166 to extend completely through the septum 112. The slit 166 may be cut using blades that may vary the inner geometry of the slit 166, so that the slit 166 may be trapezoidal instead of rectangular when viewed in a cross-sectional plane. Further, various preloads may be placed on the septum 112 during the slitting process to change the inner geometry.

The first wall 160 is stretched downward into the housing 110 when an instrument is disposed into the slit 166 to seal against leakage at an interface between the instrument and the septum 112 before, during and after extension of the instrument through the slit 166. The septum 112 may be lubricated to facilitate insertion of the instrument into the septum 112, which lubrication may be applied while forming the slit 166 or by other methods such as incorporating the lubrication into the septum material or by applying lubricous coatings to an outer surface 168 of the septum 112. The slit 166 elastically retracts upon removal of the instrument.

Features may be added to the septum 112 to keep the slit 166 in compression even after the molding process is completed. That is, during the molding process, the material of the septum 112 shrinks. As a consequence, without further steps, the slit 166 will be placed in tension, causing the slit 166 to open. As illustrated in Figs. 2-4, and perhaps best seen with reference to Fig. 4, an inner surface 170 of the septum 112 has lips 172 that depend from the inner surface 170 on either side of the slit 166. Furthermore, the septum 112 includes facets 174 depending from the lips 172 on opposite sides of the lips 172. The cooperation of the lips 172, as reinforced by the facets 174, reduces the tendency of the slit to open.

In particular, as seen in Figs. 3 and 4, the facets 174 have an angled surface 176. The angled surfaces 176 have an edge 182 along which they meet the planar, opposed surfaces 184, 186 of the lips 172. These planar, opposed surfaces 184, 186 are parallel to a longitudinal axis 188 of the connector 100 (see Fig. 3) between the interfaces with the edges 182 of the angled surfaces 176 and a proximal end 190 of the lips 172. The planar surfaces 184, 186 converge in the direction of the longitudinal axis 188 to a line 192 at runs between first and second ends 194, 196. As illustrated, neither the line 192 nor the section of the surfaces 184, 186 that leads up to the line 192 is in contact with structures of the gland 114.

However, the features that improve the ability of the slit 166 to remain closed may also have an undesirable effect on the amount of force required by the user to insert an instrument, whether Luer syringe, blunt cannula, etc., into the slit 166. To reduce the force required for insertion while biasing the slit 166 closed, additional features may be added to the septum 112, which also may best be seen in Fig. 4. The first and second ends 194, 196 of the lips 172 each have a rounded depression 198, 200 formed therein. The rounded depressions 198, 200 each define a region of reduced cross-section, thereby reducing the force required to open the slit 166 through the insertion of an instrument into the slit 166.

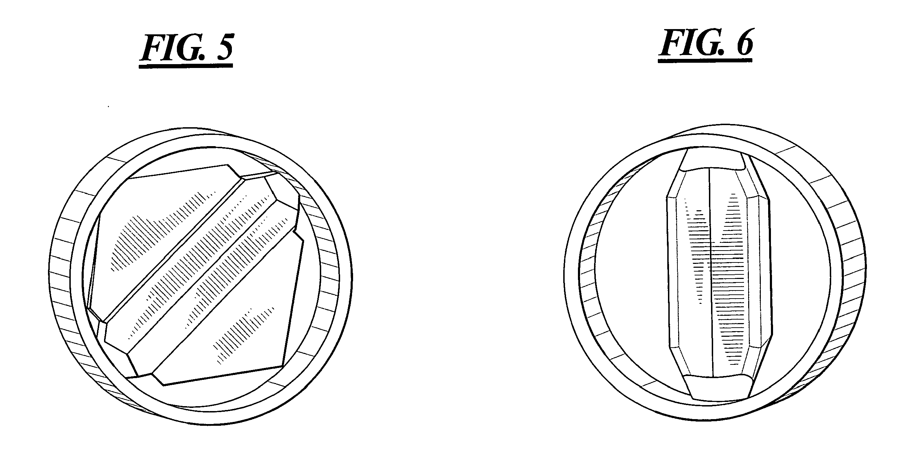

It will be recognized that it is not necessary that an embodiment of a needleless connector 100, even one using an overmolded slit septum as the valve 112, include all of the features illustrated in Figs. 2-4. In fact, the rounded depressions 198, 200 at the ends of the lips 172 may be removed, as illustrated in Fig. 5. Further, the facets 174 that support and reinforce the lips 172 may also be removed, as illustrated in Fig. 6. Thus, the septum 112 illustrated in Figs. 2-4 is only one such embodiment that may be used with the needleless connector 100 according to the present disclosure.

Following the inlet section 130 along the passage 164 in which the overmolded slit septum 112 is disposed, the passage 164 widens in cross-sectional area as it moves along from the distal end 132 to the proximal end 142, as best seen in Figs. 7 and 8. In a transition region 210, the single passage is divided into a plurality of passages 212 through the presence of a plurality of dividers 214; while eight passages 212 are illustrated, a greater or a lesser number of passages 212 may be present in a given embodiment. Each divider 214 has spaced side surfaces 216, 218, the side surfaces 216, 218 of adjacent dividers 214 defining one of the plurality of passages 212. The side surfaces 216, 218 may be angled relative to the orientation illustrated, so as to create a vortex within the connector 100 that may assist in flushing the connector 100. Each divider 214 also includes a stepped region 220 that defines a shoulder 222, the purpose of which is explained in greater detail below.

Once past the transition region 210, the plurality of passages 212 converge into a single passage 224. However, unlike the passage 164 above the transition region 210, which is circular in cross-section, this passage 224 has an annular shape. In particular, the annular passage 224 is defined by a surface 226 of the inlet section 130 and an opposing surface 228 formed at the distal end 146 of the outlet section 136.

Shifting then to the distal end 146 of the outlet section 136, as seen in Figs. 3 and 9, the distal end 146 has at least one opening 240 formed in the surface 228 in fluid communication with the annular passage 224. As illustrated, two openings 240 are provided at opposite ends 242, 244 of a cross passage 246 that passes through the distal end 146 of the outlet section 136. It will be recognized that a single cross passage 246 may be advantageous in terms of its fabrication using a molding process, but it will also be recognized that the outlet section 136 is not restricted to such an arrangement or number of openings and passages.

The cross passage 246 connects to a passage 248 disposed along a longitudinal axis of the outlet section 136, which passage 248 has at its proximal end 250 the opening 140 that defines the outlet 122. The axial passage 248 is orthogonal to the cross passage 246, forming a T-shaped intersection, although it will be recognized that the passages 246, 248 could meet at some other angle as well. In particular, the axial passage 248 may be defined by a lumen 252 of a projection 254 from the outlet section 136.

As illustrated, the projection 254 is in the form of a Luer tip, and a skirt 256 is arranged coaxially with the projection 254, and has a threaded region 258 formed on an inner surface 260 thereof. Consequently, the illustrated embodiment may be used as a Luer lock. However, the present disclosure is not limited to an outlet section 136 with such an arrangement; this is simply one embodiment according to the present disclosure. The projection 254 may be formed as a slip Luer by omitting the threaded region 258. The connector 100 could instead be formed as the injection arm of a Y-site or Y-set or as an inlet on a stopcock or manifold, or formed integrally with a catheter. In any event, the housing 110 may have indentations that facilitate gripping of the connector 100.

Returning then to Figs. 2 and 3, and comparing these with Fig. 7, disposed in the passage 164 prior to the transition region 210 is the gland 114. The gland 114 is thus disposed in the housing 110 between the inlet 120 and the outlet 122. The gland 114 functions to limit, and preferably to prevent, a retrograde flow of fluid through the outlet 122 into the housing 110 when a Luer syringe, blunt cannula, or other instrument is inserted into and then withdrawn from the connector 100. In particular, the gland 114 may provide a positive pressure to eject fluid from the outlet 122 upon withdrawal of the instrument from the connector 100.

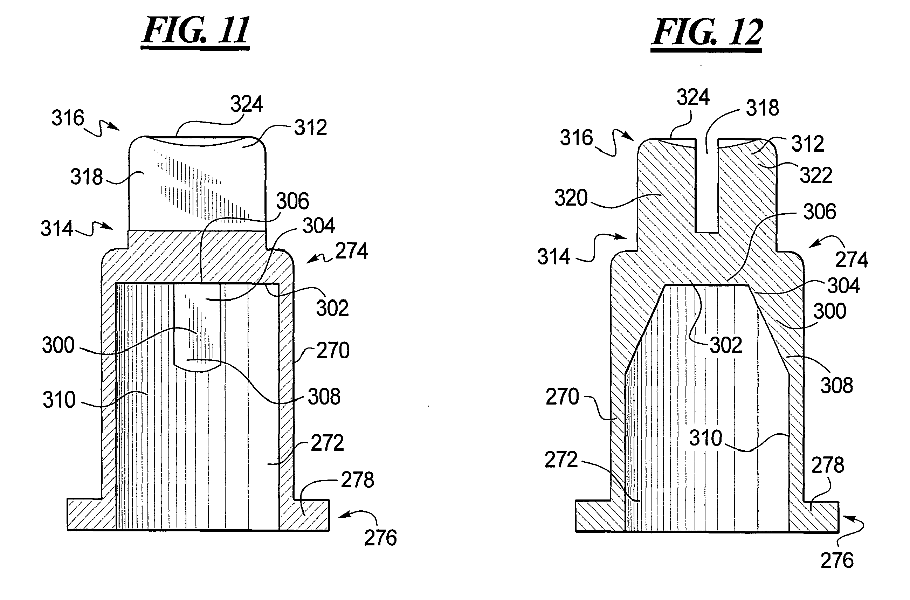

As seen in Figs. 2 and 3, and also with reference to Figs. 10-12, the gland 114 has a deformable wall 270 defining a space 272. As illustrated, the deformable wall 270 is a cup-shaped wall having a closed distal end 274 and an open proximal end 276. The wall 270 also includes a flange 278 disposed about the periphery of the open proximal end 276.

The space 272 defined by the deformable wall 270 is in communication with the at least one vent 124 formed in the housing 110 via the open proximal end 276. As illustrated, there are two vents 124, each vent 124 defined by a passage 280, 282 formed in the outlet section 136. It will be recognized that a greater or a lesser number of vents 124 may be included, and the vents 124 may be defined by structures other than the outlet section 136 as identified in the illustrated embodiment.

In addition, the outlet section 136 may include a trough 284 having a first end 286 and a second end 288. Each passage 280, 282 is connected to one of the first end 286 or the second end 288 of the trough 284. While a trough 284 is an optional feature, it may be an advantageous feature, in that the trough 284 provides a larger opening for access to the two passages 280, 282 that define the vents 124, thereby limiting the chances that the gland 114 will restrict access to the vents 124 to prevent escape of air from the space 272. It will also be recognized that the number of vents and their placement (i.e., two vents 124 spaced at opposite ends 286, 288 of the trough 284) assist in limiting the chances that the gland 114 will restrict access to the vents 124 to prevent escape of air from the space 272.

The gland 114 is maintained in place within the housing 110 with the proximal open end 276 in communication with the vents 124 at the distal end 146 of the outlet section 136 through cooperation of the inlet section 130, the outlet section 136, and the gland 114. As noted above, the dividers 214 that are formed in the transition region 210 of the inlet section 130 have a stepped shoulder 222. This shoulder 222 faces a surface 290 at the distal end 146 of the outlet section 136 (compare Figs. 7 and 9). The flange 278 of the gland 114 is disposed between the opposing surfaces of the inlet section 130 and the outlet section 136 to attach the gland 114 to the housing 110. Thus, the number of dividers 214 may be influenced by a desired flow rate, but also the need to maintain a sufficient amount of surface in the shoulder 222 so as to cooperate with the surface 290 to maintain the gland 114 in place.

As noted above, the gland 114 is deformable to limit retrograde flow into the connector 100. To control the deformation of the gland 114, the gland 114 has at least one reinforcement 300 attached to the deformable wall 270 within the space 272. The at least one reinforcement 300 causes the deformable wall 270 to collapse in a preferred fashion, with collapse occurring inwardly near the center of the gland 114, similar to a bellows or accordion. To this end, the distal end 274 of the cup-shaped wall 270 defines an internal shoulder 302, and the at least one reinforcement 300 is attached to the deformable wall 270 at the internal shoulder 302. As illustrated, the reinforcement 300 is a triangular tab attached across the internal shoulder 302, with a first end 304 formed integrally with a transverse surface 306 of the wall 270 and a second end 308 formed integrally with a lateral surface 310 of the wall 270.

It will be recognized that the shape and relative dimensions of the reinforcements 300 as illustrated are only one embodiment according to the present disclosure. It will also be recognized that other changes to the gland 114 may be made in conjunction with the reinforcements 300 to vary the collapsing profile and/or collapsed shape of the gland 114. For example, the height of the wall 270 may vary the collapsing profile, as may the thickness of the wall 270, which may vary between proximal and distal ends. In the alternative or in addition, the wall 270 may be molded in layers, each layer having a different durometer value, to vary the collapsing profile.

The collapsing profiles and/or collapsed shape may influence not only the displacement provided (e.g., positive, neutral or negative), but the profiles may influence the flow of fluid about the gland 114. Moreover, by varying the shape, the size or the placement of the reinforcement 300 or the height or the thickness of the wall 270, while maintaining the cross-section of the gland 114, a family of glands may be produced that permit use of the connector 100 in pediatric as well as adult settings.

The gland 114 also includes features that guide the contact between a Luer syringe, blunt cannula or other instrument and the gland 114. For example, the gland 114 includes a post 312 that depends from the wall 270 toward the septum 112, the post 312 having a proximal end 314 attached to the wall 270 and a free distal end 316. See Figs. 2 and 3, as well as Figs. 10-12. The post 312 has a groove 318 that extends from the distal end 316 to the proximal end 314 of the post 312, dividing the post 312 into two sections 320, 322. The groove 318 assists in maintaining an open path for passage of fluids from the instrument into the connector 100. The distal end 316 has a concave depression 324 which assists in centering the instrument on the gland 114.

As illustrated, the at least one reinforcement 300 is attached to the deformable wall 270 along an axis transverse to the groove 318. In fact, as illustrated, two reinforcements 300 are shown, the two reinforcements 300 attached to the deformable wall 270 along an axis transverse to the groove 318. As is shown in the other embodiments below, this is not an exclusive orientation of the reinforcements 300 relative to the groove 318, but one such embodiment.

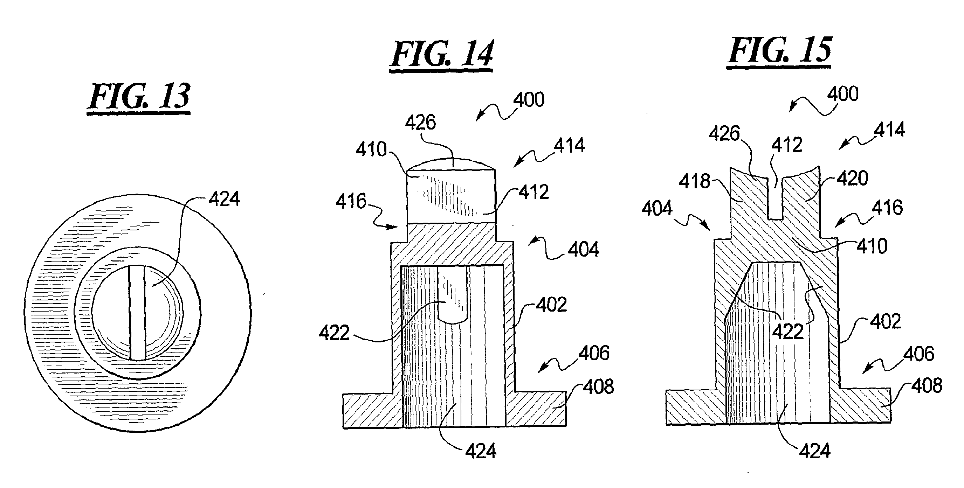

Figs. 13-15 illustrate an alternative embodiment of a gland 400 for use in a needleless connector similar to that in Figs. 1-9. In general terms, the gland 400 is substantially structurally similar to that illustrated in Figs. 10-12. For example, the gland 400 has a cup-shaped deformable wall 402 with a closed distal end 404 and an open proximal end 406, a flange 408 being disposed about the open proximal end 406. The gland 400 also has a post 410 with a groove 412 formed therein between a distal end 414 and a proximal end 416 such that the gland 400 has separate sections 418, 420. Further the gland 400 has a pair of reinforcements 422 disposed in a space 424 defined by the cup-shaped wall 402, the reinforcements 422 being disposed along an axis transverse to the axis along which the groove 412 is formed.

Where the gland 400 of Figs. 13-15 differs from the gland 114 of Figs. 10-12 relates to the distal end 414 of the post 410. In particular, the distal end 404 of the gland 400 does not have a rim or edge that bounds a concave depression 426 in the distal end 404. Instead, the depression 426 of the distal end 404 extends to the periphery of the post 410.

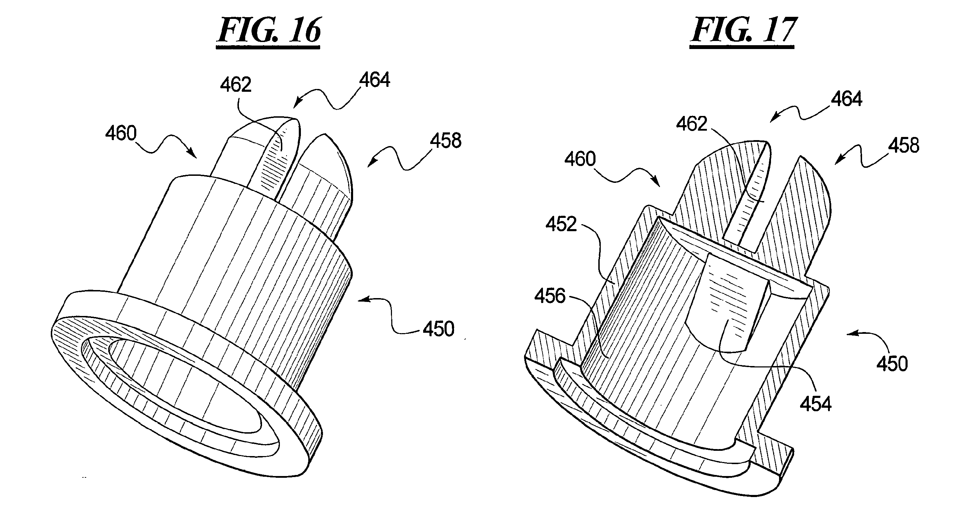

Another embodiment of a gland 450 that may be used with a needleless connector, such as is illustrated in Figs. 1-9, is illustrated in Figs. 16 and 17. Similar to the embodiments in Figs. 10-12 and 13-15, the gland 450 of Figs. 16 and 17 has a cup-shaped wall 452 with reinforcements 454 disposed in a space 456 defined by the cup-shaped wall 452. The gland 450 also has a post 458 that depends from a distal end 460 of the cup-shaped wall 452, the post 458 being separated by a groove 462 into halves. However, unlike the preceding embodiments, the post 458 does not have a distal end 464 that is concave, but rather a distal end 464 that is convex, which shape is believed to limit the possibilities that an outlet of any instrument inserted into the septum will become occluded through cooperation with the post 458. Furthermore, the reinforcements 454 are disposed along an axis that is parallel to, rather than transverse to, the axis of the groove 462.

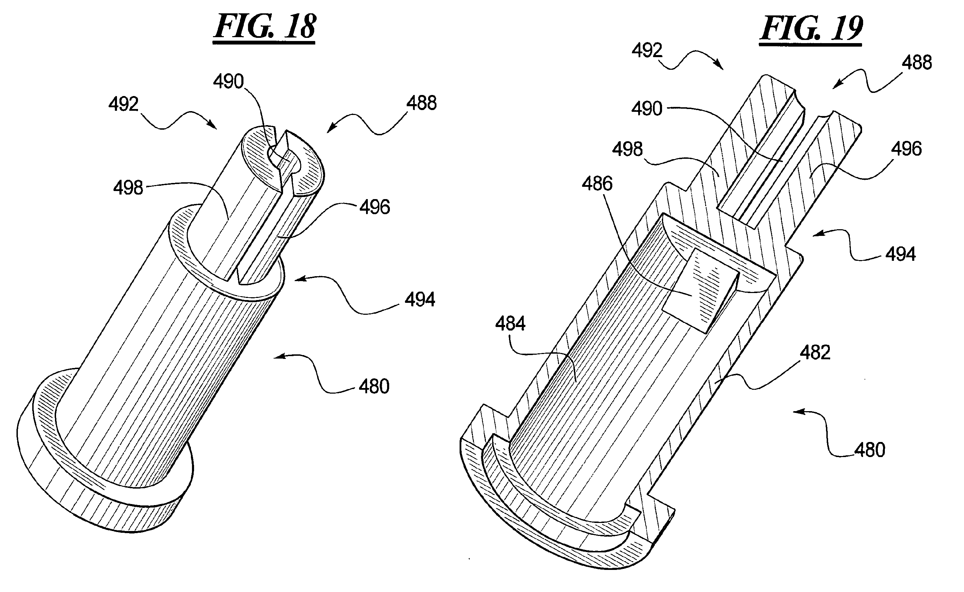

A still further embodiment of a gland 480 that may be used with a needleless connector, such as is illustrated in Figs. 1-9, is illustrated in Figs. 18 and 19. This embodiment is most closely similar to that illustrated in Figs. 16 and 17. That is, the gland 480 includes a cup-shaped wall 482 that defines a space 484 with reinforcements 486 disposed therein, and a post 488 with a groove 490 formed from a distal end 492 to a proximal end 494 to divide the post 488 into two sections 496, 498. Further, the reinforcements 486 are disposed along an axis parallel to an axis of the groove 490.

However, unlike the embodiment illustrated in Figs. 16 and 17, or that of the embodiments of Figs. 10-12 or 13-15, the groove 490 does not simply extend from the distal end 492 to the proximal end 494, so as to form a rectangular parallelepiped space between opposing surfaces on the two sections 496, 498 of the post 488. Instead, the groove 490 has a cylindrical space formed along a longitudinal axis of the gland 480. The groove 490 thus defines a space that is the union of a rectangular parallelepiped and a cylinder. It is believed that the cylindrical space will limit the chances that the distal end 492 of the post 488 will prevent passage of fluid from an instrument disposed in a connector in which the gland 480 is used, which may be particularly important as the distal end 492 of the post 488 is planar, instead of concave or convex.

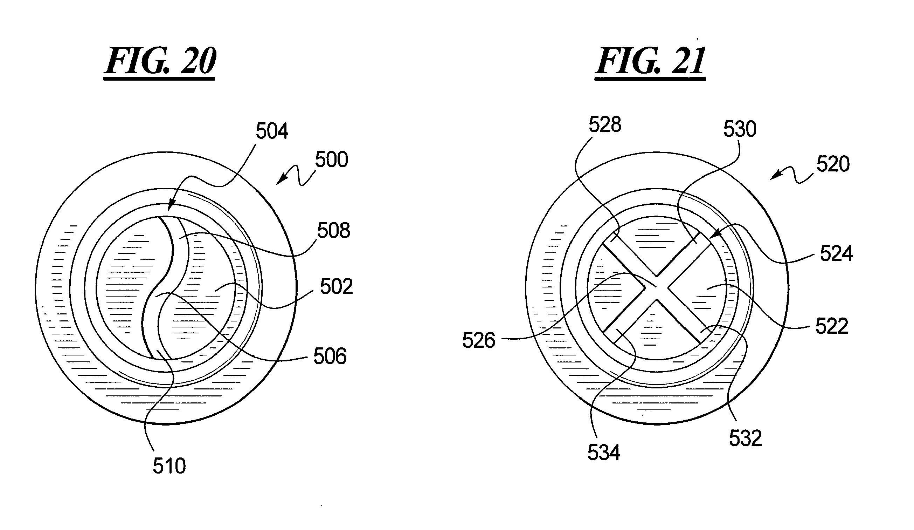

It will be recognized that further embodiments are possible wherein the groove has geometries different than those illustrated in the embodiments described above. For example, Figs. 20 and 21 illustrate other groove, or channel, geometries that may be used. As seen in Fig. 20, a gland 500 includes a post 502 with a groove 504. The groove 504 has a center 506 with outwardly extending arms 508, 510. The arms 508, 510 are curved, such that the groove 504 appears to be S-shaped when viewed from the end, as in Fig. 20. As seen in Fig. 21, a gland 520 includes a post 522 with a groove 524. The groove 524 also has a center 526 with outwardly extending arms 528, 530, 532, 534. The arms 528, 530, 532, 534 are straight, such that the groove 524 appears to be X-shaped when viewed from the end, as in Fig. 21. Such geometries may assist in the mixing of fluids during injection, or the flushing of the connector and associated catheter after injection, as may other geometries, such as V-shaped or inverted V-shaped grooves. Further, similar to the embodiment in Figs. 18 and 19, the centers 506, 526 of the grooves 504, 524 may have an enlarged cylindrical space to potential improve fluid flow.

It will also be recognized that, according to other variants, the groove may be disposed so that the groove is not aligned with the axis of the post, such non-alignment potentially having a similar benefit relative to the mixing of fluids during injection or the flushing of the connector and catheter after injection. Further modifications may be made wherein the groove does not have a consistent cross-section axially between distal and proximal ends. That is, as illustrated in the embodiments above, the groove has the same cross-section in all planes orthogonal to the axis of the post. However, it is possible to have a groove that varies in shape or orientation instead, such that the groove "twists" between the ends of the post, or has a tapering cross-section between the ends of the post.

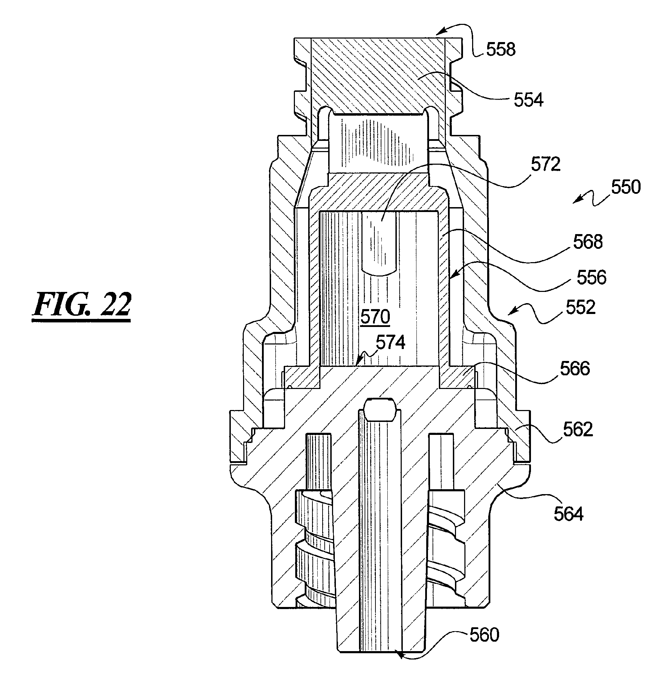

A further embodiment of a needleless connector 550 is illustrated in Fig. 22, the connector 550 being similar in many respects to the connector 100 discussed above relative to Figs. 1-4 and 7-12. That is, the connector 550 includes a housing 552, a valve 554, and a gland 556. The valve 554 and the gland 556 are disposed in the housing 552. The valve 554 controls the flow of the fluid through the housing 552 between an inlet 558 and an outlet 560, which inlet 558 and outlet 560 are formed in different sections 562, 564 of the housing 552. The gland 556 has a flange 566 that is disposed between surfaces of the sections 562, 564 to hold the gland 556 in position. The gland 556 also includes a wall 568 that defines a space 570 in which at least one reinforcement 572 is disposed.

Unlike the connector 100, the space 570 is not in communication with a vent. Instead, the space 570 is closed by a surface 574 of the section 564 of the housing 552. When an instrument contacts the gland 556, deformation of the gland 566 will occur, but the fluid (e.g., air) disposed in the space 570 is not able to escape from the space 570. Instead, the air may be compressed into a smaller space, with the wall 568 of the gland 556 collapsing as before, guided by the presence of the reinforcement 572. As such, the embodiment according to Fig. 22 may not provide a positive pressure or displacement as the connector 100, but it may instead provide a clinically neutral displacement. Even in this instance, the presence of the reinforcement 572 within the space 570 is believed to advantageously guide the deformation of the gland 556.

Use of the above-mentioned needleless connector and gland, according to any of the various embodiments described herein, may provide one or more of the following advantages relative to conventional needleless connectors, even those that include mechanisms for limiting retrograde flow. In particular, it is believed that the positioning of the reinforcements inside the space defined by the gland provides a more consistent deformation than in other devices used to provide positive pressure or positive displacement, or neutral displacement for that matter. Further, it is believed that the shape of the end of the gland provides for more consistent application of force by the instrument (Luer syringe, cannula, etc.) to the gland, while maintaining adequate flow.

Still further advantages may be obtained by impregnating the gland, the septum or the housing with an antimicrobial material or composition, or by coating the gland, the septum or the housing with such a material. For example, the antimicrobial material may include chemical disinfectants, for example, alcohols, such as isopropanol and ethanol. Biguanides may also be used, including chlorhexidine and its salts (e.g. chlorhexidine acetate, chlorhexidine gluconate, chlorhexidine hydrochloride and chlorhexidine sulfate). Further examples include, bisphenols, including triclosan, and halogen-releasing agents, including chlorine and iodine compounds. Silver and its salts (e.g. silver acetate, silver iodide, silver nitrate, silver sulfadiazine) may be included, as may copper and its salts. As a further alternative, quaternary ammonium compounds, including benzalkonium chloride, may be used. Still further exemplary alternatives include antimicrobial dyes, including acridines and crystal violet, boric acid, salicylic acid and N-halamines.

## Claims

1. A needleless connector (100) comprising:
a housing (110) having an inlet (120) and an outlet (122);
a valve (112) disposed in the housing to control flow between the inlet and the outlet; and
a gland (114) disposed in the housing between the inlet and the outlet,
the gland having a deformable wall (270) defining a space (272) and at least one reinforcement (300) attached to the deformable wall within the space.

2. The needleless connector according to claim 1, wherein the housing (110) has at least one vent (124) and the space (272) is in communication with the at least one vent.

3. The needleless connector according to claim 2, wherein the housing (110) comprises at least one passage (280, 282), the at least one passage defining the at least one vent (124).

4. The needleless connector according to claim 3, wherein the housing (110) comprises a trough (284) having a first end (286) and a second end (288), the at least one passage (280, 282) connected to one of the first end or the second end of the trough.

5. The needleless connector according to any one of claims 2 to 4, wherein the deformable wall (270) comprises a cup-shaped wall having a first, closed end (274) and a second, open end (276), the second, open end being in communication with the at least one vent (124).

6. The needleless connector according to claim 5, wherein the first, closed end (274) defines an internal shoulder (302) therein, and the reinforcement (300) is attached to the deformable wall (270) at the internal shoulder.

7. The needleless connector according to claim 6, wherein the reinforcement (300) comprises a tab attached across the internal shoulder (302), said tab optionally formed integrally with the deformable wall (270).

8. The needleless connector according to any one of claims 1 to 7, wherein the housing (110) comprises an inlet section (130) with an aperture (134) defining the inlet and an outlet section (136) with an aperture (140) defining the outlet, the inlet section attached to the outlet section with the gland (114) disposed therebetween.

9. The needleless connector according to any one of claims 5 to 7, wherein the gland (114) comprises a post (312) that depends from the first, closed end (274) toward the valve (112), the post having a first end (314) attached to the first, closed end and a second end (316).

10. The needleless connector according to claim 9, wherein the post (312) has a groove (318) that extends from the first end (314) to the second end (316) of the post, dividing the post into two sections (320, 322).

11. The needleless connector according to claim 10, wherein the at least one reinforcement (300) is attached to the deformable wall (270) along an axis transverse to the groove (318).

12. The needleless connector according to any one of claims 9 to 11, wherein the second end of the post (312) is concave or convex.

13. The needleless connector according to any one of the preceding claims, wherein the valve (112) is spaced from the gland (114).

14. The needleless connector according to any one of the preceding claims, wherein the valve (112) comprises a septum with a slit (166) therethrough.

15. The needleless connector according to claim 14, wherein the septum has an outer surface (168) and an inner surface (170), and comprises a pair of lips (172) depending from the inner surface to either side of the slit (166).

16. The needleless connector according to claim 15, wherein the septum comprises facets (174) disposed on either side of the lips (172).
